# EUROPÄISCHE PATENTSCHRIFT

(11) **EP 1 818 664 B1**
(45) Veröffentlichungstag und Bekanntmachung des Hinweises auf die Patenterteilung: **01.05.2013**
(21) Anmeldenummer: 06002802.4
(22) Anmeldetag: 13.02.2006
(51) Int. Cl.: G01N 7/00, A61M 5/168

(54) **Vorrichtung zur Erkennung einer Druckänderung im Flüssigkeitspfad einer Mikrodosiervorrichtung**
Device for detection of a change of pressure in a canal of a microdosing device
Dispositif de détection de changement de pression dans un canal de liquide dans un dispositif de microdosage

(43) Veröffentlichungstag der Anmeldung: 15.08.2007
(73) Patentinhaber: F.Hoffmann-La Roche AG, 4070 Basel (CH); Roche Diagnostics GmbH, 68305 Mannheim (DE)
(72) Erfinder: Haueter, Ulrich, 3506 Grosshöchstetten (CH); Bosshard, David, 3251 Wengi (CH); Heutschi, Ivan, 3506 Grosshöchstetten (CH); Rufer, Thomas, 3072 Ostermundigen (CH); Stoller, Hans-Peter, 3012 Bern (CH)
(74) Vertreter: Rentsch Partner AG

(56) Entgegenhaltungen:
- US-A- 4 882 575
- US-A- 5 720 721
- US-A- 5 989 222
- US-A1- 2003 167 035
- US-A1- 2005 178 206
- US-B1- 6 572 604

## Beschreibung

Die vorliegende Erfindung betrifft eine Vorrichtung zur Durchführung eines Verfahrens zur Erkennung einer Druckänderung im Flüssigkeitspfad einer Mikrodosiervorrichtung sowie eine Mikrodosiervorrichtung mit der Vorrichtung gemäss den Oberbegriffen der unabhängigen Patentansprüche.

Bei der Fein-Dosierung flüssiger Stoffe im Mikroliter-Bereich, wie sie in der pharmazeutischen und der chemischen Industrie und insbesondere auch in der Medizin zur Zuführung von flüssigen Medikamenten, z.B. Insulin, in den Körper von Patienten zur Anwendung kommt, ist es von.grösster Wichtigkeit, dass die gewünschte Dosierung exakt eingehalten wird und ein etwaiger unbeabsichtigter Zuführungsunterbruch, wie er durch ein leeres Flüssigkeitsreservoir oder eine Okklusion im Flüssigkeitspfad entstehen kann, sofort entdeckt und behoben wird.

Aus dem Stand der Technik ist bekannt, bei Insulinpumpen mit spindelgetriebenen Förderkolben das Antriebsmoment oder die Abstützkraft der Spindel zu Überwachen und aus einer Zunahme dieser Grösse auf eine Okklusion im Flüssigkeitspfad zu schliessen. Diese Art der Überwachung ist jedoch ungenau und unzuverlässig, da viele Störgrössen, wie z.B. die Reibung zwischen Förderkolben und Wandung des Flüssigkeitsreservoirs, praktisch nicht erfasst und eliminiert werden können.

Man ist deshalb dazu übergegangen, den Fluiddruck im Flüssigkeitspfad dadurch zu messen, dass ein mit dem Fluid beaufschlagtes und den Fluiddruck übertragendes oder sich unter dem Fluiddruck veränderndes Element mechanisch mit einem Sensor gekoppelt wird, welcher die von dem Element übertragene Druckkraft oder eine Bewegung desselben ermittelt zur Berechnung des Fluiddrucks. Da das mit dem Fluid in Kontakt stehende.Element in den meisten Fällen aus Hygienegründen als Einwegartikel ausgeführt ist, der Sensor aus Kostengründen jedoch mehrfach verwendet werden muss, ergibt sich bei den bekannten Mikrodosiervorrichtungen heute der Nachteil, dass die Genauigkeit der Druckmessung massgeblich von der Qualität der mechanischen Kopplung zwischen dem mit dem Fluid in Kontakt stehenden Element und dem Sensor abhängt, wodurch sich bei kostenmässig vertretbarer Tolerierung der Einwegartikel eine relativ grosse Streuung der Genauigkeit von Messung zu Messung ergibt.

Aus US 2005/0178206 A1 ist zudem eine Dosiervorrichtung bekannt, bei der Druckänderungen im Flüssigkeitspfad dadurch detektiert werden, dass die Ausdehnung eines den Flüssigkeitspfad bildenden Schlauchs berührungslos durch Projektion eines Schattenbildes des Schlauchs auf einen optischen Sensor ermittelt wird. Hierdurch werden die zuvor erwähnten Nachteile des Standes der Technik zumindest teilweise vermieden.

Es stellt sich die Aufgabe, alternative Vorrichtungen zu letztgenanntem Stand der Technik zur Verfügung zu stellen, welche die zuvor genannten Nachteile des Standes der Technik nicht aufweisen oder diese zumindest teilweise vermeiden.

Diese Aufgabe wird durch die Vorrichtung zur Durchführung eines Verfahrens zur Erkennung einer Druckänderung im Flüssigkeitspfad einer Mikrodosiervorrichtung und die Mikrodosiervorrichtung gemäss den unabhängigen Patentansprüchen gelöst.

Bei dem Verfahren zur Erkennung einer Druckänderung, wie beispielsweise eines Druckanstiegs oder eines Druckabfalls im Flüssigkeitspfad einer Mikrodosiervorrichtung für flüssige Stoffe, wie z.B. eines Infusionsgerätes zum Zuführen von flüssigen Medikamenten in den Körper einer Person, zu dessen Durchführung die Vorrichtung geeignet ist, wird in einem ersten Schritt eine Körperlichkeit bereitgestellt, welche derartig ausgestaltet ist, dass sie in einem Innenraum mit einer unter Druck stehenden Flüssigkeit beaufschlagt werden kann und bei Änderung des Druckes der Flüssigkeit eine Gestaltänderung erfährt. Diese Körperlichkeit wird mit der im Flüssigkeitspfad einer Mikrodosiervorrichtung geführten Flüssigkeit in Wirkverbindung gebracht, d.h. in ihrem Innenraum direkt mit der im Flüssigkeitspfad geführten Flüssigkeit beaufschlagt oder indirekt mit einer mit der Flüssigkeit im Flüssigkeitspfad, z.B. über eine Membran oder einen Kolben, druckmässig wirkverbundene identischen Flüssigkeit oder nicht identischen Hilfs-Flüssigkeit beaufschlagt, wobei als Hilfs-Flüssigkeit z.B. Silikonöl dienen kann. Dabei kann die erstgenannte, direkte Beaufschlagung durch Einkoppeln der Körperlichkeit "Inline" in den Flüssigkeitspfad, in einen Bypass zum Flüssigkeitspfad oder in einen vom Flüssigkeitspfad abzweigenden Blindkanal erfolgen, während die letztgenannte Möglichkeit zweckmässigerweise als Blindkanalausführung ausgebildet wird. Sodann wird in einem weiteren Schritt eine durch Druckänderung der Flüssigkeit im Flüssigkeitspfad hervorgerufene Gestaltänderung der Körperlichkeit mittels berührungsloser Messverfahren detektiert, zur Erkennung der Druckänderung der Flüssigkeit im Flüssigkeitspfad.

Dabei wird zum Detektieren der Gestaltänderung ein Lichtstrahl an einer sich bei Druckänderung verlagernden und/oder verformenden Fläche reflektiert.

Die hierfür anwendbaren Messverfahren bzw. die hierfür verwendbaren kommerziell erhältlichen berührungslosen Messmittel sind dem Fachmann bekannt und werden hier deshalb nur beispielhaft und im Zusammenhang mit bevorzugten Ausführungsformen der Erfindung aufgezeigt.

Das Verfahren weist den Vorteil auf, dass die funktionelle Anbindung zwischen der mit der Flüssigkeit im Flüssigkeitspfad in Wirkverbindung stehenden Körperlichkeit und den Messmitteln ohne mechanische Interaktion erfolgt, so dass sich die Messungenauigkeiten, wie sie aus dem Stand der Technik insbesondere für den Fall bekannt sind, dass die Messmittel mehrfach verwendet werden während die mit der Flüssigkeit in Kontakt stehende Körperlichkeit als Einwegartikel regelmässig gewechselt wird, deutlich verringert werden können.

Unter einer Mikro-Dosiervorrichtung werden hier Dosiervorrichtungen verstanden, welche in der Lage sind, Flüssigkeiten im Mikroliterbereich zu dosieren.

In einer bevorzugten Ausführungsform des Verfahrens wird der reflektierte Lichtstrahl mit einem photosensitiven Sensor, bevorzugterweise mit einem Punkt-, Linien- oder X-Y-Sensor (5) empfangen.

In einer weiteren bevorzugten Ausführungsform des Verfahrens erfolgt ein mehrfacher Wechsel der mit der Flüssigkeit im Flüssigkeitspfad in Wirkverbindung stehenden Körperlichkeit bei gleichzeitiger mehrfacher Verwendung derselben Messmittel. Dabei wird nach jedem Wechsel der Körperlichkeit zuerst die Gestalt derselben in einem bestimmten, definierten Referenzzustand, bevorzugterweise in einem Betriebzustand, in welchem der Flüssigkeitspfad druck- bzw. unterdrucklos ist, ermittelt. Sodann wird zur Erkennung einer Druckänderung die relative Gestaltänderung gegenüber dieser Referenzgestalt detektiert. Es erfolgt also ein "Nullabgleich" mit anschliessenden Relativmessungen, wodurch sich der individuelle "Offset" oder mit anderen Worten die individuelle Abweichung der jeweiligen gerade verwendeten Körperlichkeit als Fehlergrösse eliminieren lässt.

In noch einer weiteren bevorzugten Ausführungsform des Verfahrens wird eine Dehnung oder Schrumpfung der mit der Flüssigkeit im Flüssigkeitspfad in Wirkverbindung stehenden Körperlichkeit detektiert, und zwar bevorzugterweise eine Durchmesser- oder Volumenänderung eines elastischen Schlauchabschnitts oder eines elastischen blasenartigen Körpers. Auf diese Weise werden kostengünstige Lösungen möglich, da die Körperlichkeit direkt von eine Schlauchleitung, welche Teil des Flüssigkeitpfades ist, gebildet werden kann.

In einer weiteren bevorzugten Ausführungsform des Verfahrens wird eine zu- oder abnehmende Verwölbung einer Fläche der mit der Flüssigkeit im Flüssigkeitspfad in Wirkverbindung stehenden Körperlichkeit detektiert, wobei bevorzugterweise die Verwölbung einer Membran detektiert wird, welche auf einer ihrer Seiten direkt mit der im Flüssigkeitspfad geführten Flüssigkeit beaufschlagt ist.

In noch einer weiteren bevorzugten Ausführungsform des Verfahrens wird eine Verschiebung, Verdrehung und/oder Neigung eines Bauteils der mit der Flüssigkeit im Flüssigkeitspfad in Wirkverbindung stehenden Körperlichkeit detektiert, und zwar bevorzugterweise eines direkt mit der im Flüssigkeitspfad geführten Flüssigkeit beaufschlagten Bauteils.

Insbesondere bei den beiden letztgenannten Ausführungsformen ist es bevorzugt, die Verwölbung der Membran bzw. die Verschiebung, Verdrehung und/oder Neigung des Bauteils mittels Reflexion eines Lichtstrahls an einer auf der Membran angeordneten Spiegelfläche auf einen photosensitiven Sensor zu detektieren, wobei für den Fall, dass als Endergebnis lediglich die Über- oder Unterschreitung eines Schwellwertes festgestellt werden soll, ein Punktsensor ausreichend ist. Soll hingegen eine Relativmessung mit vorherigem "Nullabgleich" und/oder eine differenziertere Messung erfolgen, so ist die Verwendung von Linien- oder X-Y-Sensoren geboten. Hierdurch ergibt sich der Vorteil, dass sich auch geringe Druckänderungen sofort und relativ genau erkennen lassen.

Je nach Anwendung des Verfahrens ist es zudem bevorzugt, die Körperlichkeit kontinuierlich, intervallweise oder ereignisgesteuert, z.B. jeweils nur während der Förderzeit der Dosierpumpe der Mikrodosiervorrichtung, auf eine durch Druckänderung der Flüssigkeit im Flüssigkeitspfad bedingte Gestaltänderung zu überwachen, wobei sich die intervallweise und die ereignisgesteuerte Überwachung besonders gut für batteriebetriebene Mikrodosiervorrichtungen eignen, da auf diese Weise relativ wenig Energie benötigt wird.

In einer weiteren bevorzugten Ausführungsform des Verfahrens wird mittels einer Auswertungselektronik die detektierte Gestaltänderung bzw. ein aus dieser berechneter Druckwert mit einem oder mehreren Vorgabewerten verglichen und bei Unter- oder Überschreitung eines Vorgabewertes ein akustischer, optischer und/oder taktiler Alarm ausgesandt. Dies ist insbesondere bei automatisierten Mikrodosiervorrichtungen für Medikamente wichtig, da hier etwaige durch Betriebsstörungen hervorgerufene Druckänderungen im Flüssigkeitspfad fatale Folgen haben können und sofort erkannt werden müssen.

Auch ist es vorgesehen, mittels einer Auswertungselektronik die detektierte Gestaltänderung bzw. einen aus dieser berechneten Druckwert, bevorzugterweise vor einem Vergleichen desselben mit einem Vorgabewert, um den Einfluss bestimmter Störgrössen, wie z.B. den Einfluss des Umgebungsluftrucks, der Umgebungstemperatur, der mechanischen Position der Körperlichkeit gegenüber den Messmitteln und/oder den Einfluss von Vibrationen, zu korrigieren. Hierdurch kann die Messgenauigkeit deutlich gesteigert werden.

Bevorzugterweise wird das Verfahren zum Erkennen einer Okklusion im Flüssigkeitspfad einer Dosiervorrichtung für flüssige Medikamente, bevorzugterweise im Flüssigkeitspfad einer automatischen Insulinpumpe, angewendet. Bei solchen Anwendungen kommen die Vorteile des Verfahrens besonders deutlich zum Tragen.

Ein erster Aspekt der Erfindung betrifft eine Vorrichtung zur Durchführung des zuvor beschriebenen Verfahrens. Die Vorrichtung umfasst eine Körperlichkeit mit einem flüssigkeitsdichten Innenraum mit mindestens einer Öffnung, mittels welcher der Innenraum mit dem Flüssigkeitspfad einer Mikrodosiervorrichtung derartig verbunden oder verbindbar ist, dass der Innenraum mit dem jeweiligen im Flüssigkeitspfad herrschenden oder einem von diesem abhängigen Fluiddruck beaufschlagt bzw. beaufschlagbar ist. Dabei kommuniziert der Innenraum entweder direkt mit dem Flüssigkeitspfad der Mikrodosiervorrichtung oder ist mit einem Fluidvolumen identischer oder nichtidentischer Art beaufschlagt, welches z.B. über eine Membran oder einen Kolben druckmässig mit der im Flüssigkeitspfad geführten Flüssigkeit kommuniziert. Die Körperlichkeit ist derartig ausgebildet, dass sie bei einer Änderung des Fluiddruckes in ihrem Innenraum eine Gestaltänderung erfährt. Zudem weist die Vorrichtung Messmittel auf, welche derartig ausgestaltet und mit der sich bei Druckänderung gestaltsmässig verändernden Körperlichkeit gekoppelt sind, dass mit ihnen eine Gestaltänderung detektierbar ist, ohne die Körperlichkeit zu berühren. Hierdurch lassen sich Vorrichtungen zur Verfügung stellen, welche eine einfache und robuste Ankopplung zwischen der mit der Flüssigkeit im Flüssigkeitspfad wirkverbundenen Körperlichkeit und den Messmitteln realisieren und so eine gute Messgenauigkeit auch bei als Einwegartikel ausgeführten Körperlichkeiten in Kombination mit mehrfach verwendbaren Messmitteln ermöglichen.

Dabei sind die Messmittel ausgestaltet zum Detektieren einer Lage- oder Formänderung einer sich bei Druckänderung verlagernden und/oder verformenden Fläche der Körperlichkeit mittels Reflexion eines von einem Emitter ausgesendeten Lichtstrahls an einer sich verlagernden oder verformenden reflektierenden Fläche auf einen photosensitiven Sensor, bevorzugterweise einen Punkt-, Linien- oder X-Y-Sensor.

Hierdurch ergibt sich insbesondere bei Ausführungsformen, bei denen eine Membran das unter Druckänderung seine Gestalt ändernde Element darstellt, der Vorteil, dass sowohl durch die Positionierung des Reflektors, z.B. auf der Membran (zentrisch, exzentrisch, näher oder weiter vom Emitter/Sensor entfernt), wie auch durch die Neigung des Reflektors und dessen Form (ein- oder mehrdimensional konvex, konkav, "varilux", etc.) die Eigenschaften des Systems, insbesondere betreffend Sensitivität oder Nichtlinearität, eingestellt werden können.

Bei Vorrichtungen, deren mit der im Flüssigkeitspfad geführten Flüssigkeit wirkverbundene Körperlichkeit über eine sich bei Druckänderung mehr oder weniger verwölbende Membran verfügt, wird die reflektierende Fläche bevorzugterweise als reflektierender Bereich der Membran oder als von der Membran getragener, im wesentlichen starrer Reflektor ausgebildet, wobei es zudem bevorzugt ist, den Bereich der Membran, welcher die eigentliche Reflektorfläche umgibt, satiniert auszubilden, um Streulicht und Mehrfachreflexionen zu vermeiden.

Auch ist es in diesem Zusammenhang vorteilhaft, die Membran und bevorzugterweise auch die die Membran tragende Körperlichkeit aus einem lichtundurchlässigen Material herzustellen, um einen störenden Lichteinfall von aussen zu verhindern.

In einer weiteren bevorzugten Ausführungsform der Vorrichtung ist der Innenraum der Körperlichkeit ein Teil des Flüssigkeitspfades oder bildet ganz oder teilweise eine Bypassleitung zum Flüssigkeitspfad oder einen mit dem Flüssigkeitspfad kommunizierenden Blindkanal. Durch diese direkte Beaufschlagung der Körperlichkeit wird eine praktisch verzögerungsfreie und feinfühlige Erkennung einer Druckänderung im Flüssigkeitspfad möglich.

Wird die Vorrichtung derartig ausgestaltet, dass die mit der Flüssigkeit im Flüssigkeitspfad wirkverbundene Körperlichkeit als Wechselteil ausgebildet ist, welches ohne Werkzeuge ausgetauscht werden kann, während die Messmittel an der Vorrichtung verbleiten, was bevorzugt ist, so wird ein wirtschaftlicher Betrieb der Vorrichtung auch im Medizinalbereich möglich, da hier die mit der Flüssigkeit im Flüssigkeitspfad in Verbindung stehenden Elemente aus Gründen der Hygiene regelmässig ersetzt werden müssen. Hierzu wird zweckmässigerweise die Körperlichkeit als günstig herzustellender Einwegartikel ausgebildet, während die kostenintensiven Messmittel, welche keinen Kontakt zur Flüssigkeit haben und von daher hygienetechnisch unbedenklich sind, mehrfach benutzt werden.

Bei einer bevorzugten Ausführungsform der erfindungsgemässen Vorrichtung umfasst die mit der Flüssigkeit im Flüssigkeitspfad wirkverbundene Körperlichkeit einen elastischen Schlauchabschnitt oder einen elastischen blasenartigen Körper, welcher sich bei einer Änderung des Drucks des in seinem Innenraum befindlichen Fluids bevorzugterweise im Durchmesser ausdehnt bzw. schrumpft, wobei die Messmittel dazu ausgestaltet sind, diese Dehnung oder Schrumpfung zu detektieren zur Erkennung der Druckänderung. Solche Körperlichkeiten lassen sich besonders kostengünstig zur Verfügung stellen, z.B. in Form eines elastischen Schlauchabschnitts, welcher den Flüssigkeitspfad bildet. Dabe ist es beispielweise denkbar, zur Detektion der Dehnung oder Schrumpfung einen Lichtstrahl an einem auf der dehnbaren Oberfläche angeordneten Reflektor zu reflektieren oder die Distanz zu einem Referenzelement zu messen. Geeignete Messverfahren sind dem Fachmann bekannt und wurden beispielhaft auch schon anlässlich des ersten Aspektes der Erfindung erwähnt.

In einer weiteren bevorzugten Ausführungsform der Vorrichtung umfasst die mit der Flüssigkeit im Flüssigkeitspfad wirkverbundene Körperlichkeit eine Membran, welche sich bei einer Änderung des Drucks des in seinem Innenraum befindlichen Fluids mehr oder weniger stark verwölbt. Dabei ist die Membran mit Vorteil auf einer ihrer beiden Seiten direkt mit dem Fluid beaufschlagt oder beaufschlagbar. Die Verwendung einer Membran als gestaltveränderndes Bauteil ergibt den Vorteil, dass sowohl durch geeignete Wahl der Membrangeometrie (Form, Dicke) als auch durch geeignete Materialwahl (Elastizität) die Eigenschaften des Systems in weiten Grenzen auf einfache Weise eingestellt werden können, z.B. um eine maximale Sensitivität bei minimaler Volumenänderung zu erhalten. Zudem kann bei Vorrichtungen mit mehrfach verwendbaren Messmitteln und kostengünstigen, als Einwegartikel ausgebildeten Körperlichkeiten eine gute Genauigkeit erreicht werden, da die Herstellung von Membranen mit eng tolerierten physikalischen Eigenschaften relativ einfach ist, so dass der Unterschied der Verformungscharakteristik von Membran zu Membran und daher die Abweichungen der Gestaltänderungscharakteristik von Einwegkörperlichkeit zu Einwegkörperlichkeit vernachlässigbar ist und lediglich der individuelle "Offset" der einzelnen Einwegkörperlichkeit infolge Fertigungs- bzw. Positioniertoleranzen zu nennenswerten Abweichungen führen kann, welche jedoch durch einen Nullabgleich bzw. durch Relativmessung eliminierbar sind.

Um die zuvor erwähnten Toleranzen möglichst gering zu halten, ist es zudem bevorzugt, die mit der Flüssigkeit im Flüssigkeitspfad wirkverbundene Körperlichkeit derartig auszugestalten, dass die Membran in einem Zustand, in welchem die Flüssigkeit im Flüssigkeitskanal drucklos ist oder unter einem Unterdruck steht, auf ihrer flüssigkeitsbeaufschlagten Seite an einer Kontaktfläche anliegt. Dabei ist es vorteilhaft, wenn diese Kontaktfläche mit einer Oberflächenstruktur versehen ist, welche z.B. Kerben, Rippen oder ein Pyramidenmuster aufweist, da hierdurch bei einem Druckanstieg sofort die gesamte Membranfläche druckbeaufschlagt wird und die Wahrscheinlichkeit eines Anhaftens der Membran an der Kontaktfläche deutlich verringert wird. Auch kann es in diesem Zusammenhang sinnvoll sein, zumindest die Kontaktfläche oder den gesamten Innenraum der Körperlichkeit mit einer geeigneten Antihaftbeschichtung zu versehen.

In noch einer weiteren bevorzugten Ausführungsform der Vorrichtung umfasst die mit der Flüssigkeit im Flüssigkeitspfad wirkverbundene Körperlichkeit ein starres Bauteil, welches sich bei einer Änderung des Druckes in seinem Innenraum gegenüber einem anderen Element der Körperlichkeit verschiebt, verdreht oder neigt. Ein solches Element kann beispielsweise der Kolben einer Kolben-Zylinder-Anordnung sein, welcher bevorzugterweise federkraftbelastet ist und sich bei einer Zunahme des Flüssigkeitsdrucks entgegen der Federkraft im Zylinder verschiebt, oder ein kolben- oder plattenförmiges Bauteil, welches durch ein beispielsweise sickenförmiges Element verschiebbar und bevorzugterweise federkraftbelastet geführt ist und bei Änderung des Fluiddrucks im Innenraum der Körperlichkeit eine Verschiebung oder Schrägstellung erfährt. Mit solchen Ausgestaltungen lassen sich relativ grosse Gestaltänderungen bei kleinen Druckänderungen hervorrufen, welche bevorzugterweise mittels der bereits zuvor erwähnten Distanzmessverfahren oder Reflexionsmessverfahren detektiert werden.

Dabei ist es von Vorteil, wenn der Kolben oder das kolben- oder plattenförmige Element auf einer Seite direkt mit der im Flüssigkeitspfad geführten Flüssigkeit beaufschlagt ist, da auf diese Weise eine Druckänderung direkt und praktisch verlustfrei in eine Gestaltänderung umgesetzt werden kann, so dass auch geringe Druckänderungen sofort und mit guter Genauigkeit detektiert werden können.

Je nach Anwendungsfall ist es zudem bevorzugt, wenn die Messmittel entweder zum kontinuierlichen, intervallweisen oder ereignisgesteuerten Detektieren der Gestaltänderung ausgestaltet sind bzw. ein entsprechender Betriebsmodus wahlweise eingestellt werden kann. Während eine kontinuierliche Überwachung aus messtechnischer Sicht den Idealfall darstellt, welcher jedoch eine entsprechende Energieversorgung voraussetzt und daher eher bei stationären Vorrichtungen zum Einsatz kommt, stellt eine intervallweise bzw. ereignisgesteuerte Überwachung speziell bei batteriebetriebenen, mobilen Geräten eine gute Möglichkeit dar, eine ausreichend "feinmaschige" Überwachung bei akzeptabler Batterielebensdauer zu erzielen. So ist es beispielsweise bei mobilen Geräten bevorzugt, nur z.B. etwa alle 10 Sekunden und/oder nur dann eine Messung vorzunehmen, wenn eine Förderung von Flüssigkeit im Flüssigkeitspfad erfolgt.

Weitere bevorzugte Ausführungsformen der Vorrichtung verfügen über eine Auswertungselektronik, mit welcher z.B. eine detektierte Gestaltänderung oder ein aus dieser Gestaltänderung berechneter Druckwert mit mindestens einem bevorzugterweise benutzerprogrammierbaren Vorgabewert verglichen werden kann und bei Über - oder Unterschreiten des Vorgabewertes ein Alarm ausgelöst werden kann, welcher bevorzugterweise akustisch, optisch und/oder taktil wahrnehmbar gemacht werden kann. Hierdurch wird es möglich, eine automatisierte Überwachung des Flüssigkeitspfades von Mikrodosiervorrichtungen, z.B. auf Okklusionen, vorzunehmen.

Auch ist es bevorzugt, wenn die Auswertungselektronik zudem oder alternativ in der Lage ist, bevorzugterweise vor dem Vergleichen der ermittelten Gestaltänderung oder des errechneten Druckwerts mit einem Vorgabewert, diesen mittels eines Korrekturalgorithmus um den Einfluss bestimmter Störgrössen, bevorzugterweise um den Einfluss des Umgebungsluftdruckes, der Umgebungstemperatur, mechanischer Positionsabweichungen zwischen der Körperlichkeit und den Messmitteln und/oder um den Einfluss von Vibrationen zu korrigieren. Hierdurch lässt sich die Messgenauigkeit bzw. Zuverlässigkeit der Vorrichtung deutlich steigern.

In einer bevorzugten Ausführungsform ist die Vorrichtung als eigenständige Funktionseinheit ausgebildet, welche zwecks Überwachung des Flüssigkeitspfades einer Mikrodosiervorrichtung in den Flüssigkeitspfad derselben einkoppelbar ist, was bei Mikrodosiervorrichtungen für flüssige Medikamente, insbesondere bei Insulinpumpen, bevorzugterweise mittels Standard-Luer erfolgen kann.

In einer anderen bevorzugten Ausführungsform ist die Vorrichtung integrierter Bestandteil einer Mikro-dosiervorrichtung, bevorzugterweise für flüssige Medikamente, wie z.B. einer Insulinpumpe zur zeitlich gesteuerten Abgabe von Insulin an einen Patienten, wobei es bevorzugt ist, wenn die Messmittel zur Mehrfachverwendung fest und werkzeuglos untrennbar mit dem Gehäuse der Mikrodosiervorrichtung verbunden sind und die Körperlichkeit, welche mit der im Flüssigkeitspfad geführten Flüssigkeit wirkverbunden ist und bei einer Druckänderung derselben eine Gestaltänderung erfährt, als werkzeuglos austauschbares Einwegteil ausgestaltet ist.

Ein zweiter Aspekt der Erfindung betrifft eine Mikrodosiervorrichtung, insbesondere zur zeitlich gesteuerten Abgabe von flüssigen Medikamenten, welche eine Vorrichtung gemäss dem ersten Aspekt der Erfindung umfasst, und bei welcher die mit der im Flüssigkeitspfad geführten Flüssigkeit wirkverbundene und bei Druckänderung dieser Flüssigkeit eine Gestaltänderung erfahrende Körperlichkeit als werkzeuglos austauschbares Wechselteil ausgebildet ist, während die Messmittel zum Detektieren der Gestaltänderung werkzeuglos unentfernbar an der Mikrodosiervorrichtung angeordnet sind.

Bevorzugte Ausführungen solcher Mikrodosiervorrichtungen sind bevorzugterweise tragbare Insulinpumpen zur zeitlich gesteuerten Abgabe von Insulin in den Körper einer Person, bei denen die Messmittel zur Mehrfachverwendung in das Pumpengehäuse integriert sind und die Körperlichkeit zur Einmalverwendung einen Teil des Infusionssets bzw. des Infusionsset-Adapters bildet.

Die Verwendung der Vorrichtung gemäss dem ersten Aspekt der Erfindung oder der Mikrodosiervorrichtung gemäss dem zweiten Aspekt der Erfindung zum Abgeben von flüssigen Medikamenten, insbesondere von Insulin, ist bevorzugt.

Bei solchen Mikrodosiervorrichtungen und Verwendungen kommen die Vorteile der Erfindung besonders deutlich zum Tragen.

Weitere bevorzugte Ausführungen der Erfindung ergeben sich aus den abhängigen Ansprüchen und aus der nun folgenden Beschreibung anhand der Figuren, wobei die Figuren 6, 7 und 9a-9c keine erfindungsgemässen Vorrichtungen zeigen. Dabei zeigen:
Fig. 1 eine erfindungsgemässe Mikrodosiervorrichtung in der Seitenansicht;
Fig. 2a den oberen Teil der Mikrodosiervorrichtung aus Fig. 1 mit einer ersten Ausführungsform der erfindungsgemässen Vorrichtung teilweise im Schnitt;
Fig. 2b eine Ansicht der Lage der Elemente des in Fig. 2a dargestellten oberen Teils aus Sicht der durch die Linie A-A in Fig. 2a definierten Schnittebene;
die Figuren 3a und 3b jeweils eine Darstellung wie Fig. 2a mit einer zweiten Ausführungsform der erfindungsgemässen Vorrichtung, einmal mit drucklosem und einmal mit druckbeaufschlagtem Flüssigkeitspfad;
die Figuren 4a und 4b jeweils eine Darstellung wie Fig. 2a mit einer dritten Ausführungsform der erfindungsgemässen Vorrichtung, einmal mit drucklosem und einmal mit druckbeaufschlagtem Flüssigkeitspfad;
die Figuren 5a und 5b jeweils eine Darstellung wie Fig. 2a mit einer vierten Ausführungsform der erfindungsgemässen Vorrichtung, einmal mit drucklosem und einmal mit druckbeaufschlagtem Flüssigkeitspfad;
Fig. 6 eine Darstellung wie Fig. 2a mit einer fünften, nicht erfindungsgemässen Ausführungsform der Vorrichtung;
Fig. 7 eine Darstellung wie Fig. 2a mit einer sechsten, nicht erfindungsgemässen Ausführungsform der Vorrichtung;
Fig. 8 eine Darstellung wie Fig. 2a mit einer siebten Ausführungsform der erfindungsgemässen Vorrichtung;
Fig. 9a einen Längsschnitt durch eine achte, nicht erfindungsgemässe Ausführungsform der Vorrichtung, und
die Figuren 9b und 9c jeweils einen Schnitt entlang der Linie B-B in Fig. 9a, einmal mit drucklosem und einmal mit druckbeaufschlagtem Flüssigkeitspfad.

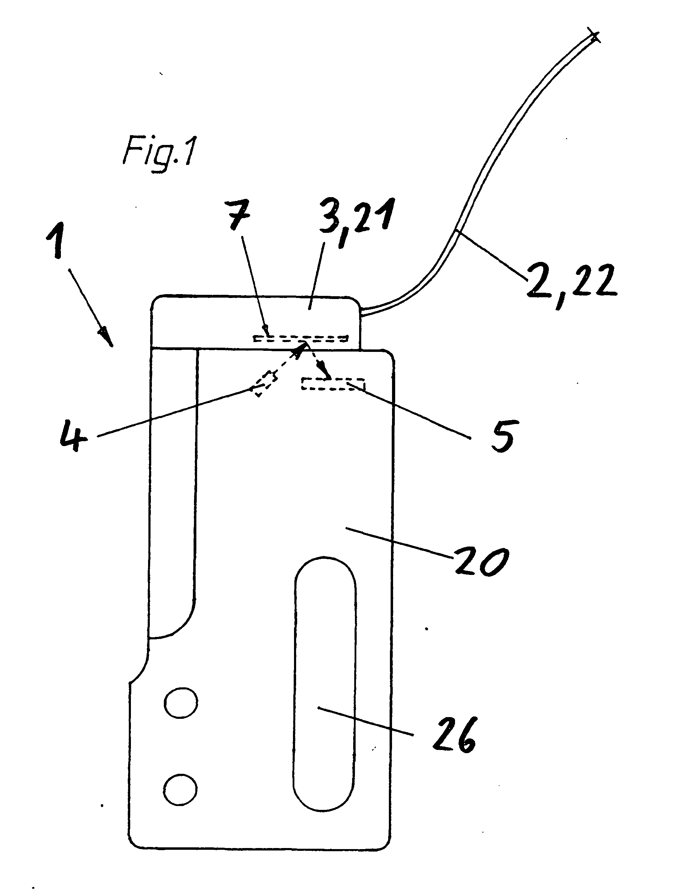

Der Grundaufbau einer erfindungsgemässen Mikrodosiervorrichtung ist aus Fig. 1 erkennbar, welche eine tragbare Insulinpumpe 1 zur zeitgesteuerten Zuführung von Insulin über ein Infusionsset 21, 22 zum Körper eines Patienten in der Seitenansicht zeigt. Das Infusionsset 21, 22 ist als Einwegartikel ausgestaltet und umfasst einen Adapterteil 21 und einen Katheter 22, welche den patientenseitigen Flüssigkeitspfad 2 der Insulinpumpe 1 bilden. Der Adapter 21 umfasst in seinem Innern eine mit der Flüssigkeit im Flüssigkeitspfad 2 beaufschlagte Kunststoffmembran 7 (gestrichelt dargestellt), welche sich bei einem Anstieg des Flüssigkeitsdrucks im Flüssigkeitspfad 2, z.B. infolge einer Okklusion im Katheter 22, in Richtung zum Pumpengehäuse 20 der Infusionspumpe 1 hin verwölbt, und bildet somit eine anspruchsgemässe Körperlichkeit 3, welche bei Änderung des Flüssigkeitsdruckes im Flüssigkeitspfad 2 eine Gestaltänderung erfährt. Zur Detektion der Gestaltänderung durch Verwölbung der Membran 7 weist die Insulinpumpe 1 angeordnet im Pumpengehäuse 20 einen Signalemitter 4 (gestrichelt dargestellt), im vorliegenden Fall eine Lichtquelle 4 mit einer Laserdiode auf, deren Lichtstrahl auf die Membran 7 auftrifft und von dieser auf einen Sensor 5 (gestrichelt dargestellt), im vorliegenden Fall einen photosensitiven X-Y-Sensor 5, reflektiert wird. Der Sensor 5 ist mit einer im Pumpengehäuse 20 befindlichen elektronischen Auswertungsschaltung (nicht gezeigt) verbunden, mittels derer aus dem Ort des Auftreffens des Lichtstrahls auf den Sensor 5 bzw. aus einer Veränderung dieses Ortes die Verwölbung der Membran 7 detektiert werden kann, zur Erkennung von Drücken bzw. Druckänderungen im Flüssigkeitspfad 2.

Des Weiteren verfügt die Insulinpumpe 1 über Anzeigemittel in Form eines Displays 26 und über einen akustischen Signalgeber, mittels welchen ein unzulässiger Druck oder eine unzulässige Druckänderung, welcher oder welche einen in der Insulinpumpe 1 abgespeicherten zulässigen Wert überschreitet, als Alarm gemeldet wird. Der Alarmlevel ist im vorliegenden Fall programmierbar.

Da es sich bei der Insulinpumpe um ein batteriebetriebenes Gerät handelt, erfolgt die Messung im vorliegenden Fall ereignisgesteuert, und zwar derart, dass lediglich bei Insulinförderung geprüft wird, ob die Membran 7 eine Verwölbung aufweist. Hierdurch lässt sich die Lebensdauer der Batterien deutlich erhöhen.

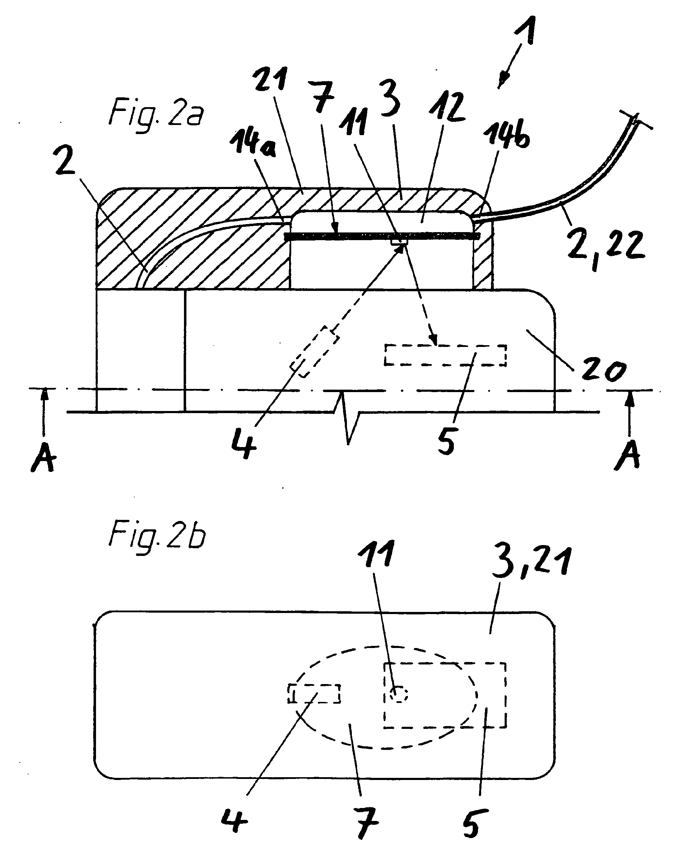

Wie aus Fig. 2a hervorgeht, welche eine Ansicht des oberen Teils der Insulinpumpe 1 mit dem Adapter 21 im Längsschnitt und bei drucklosem Flüssigkeitspfad zeigt, ist die Membran 7 derartig im Gehäuse des Adapters 21 angeordnet, dass der Flüssigkeitspfad 2 durch einen hinter der Membran 7 gebildeten Innenraum 12 führt, so dass die Membran 7 auf ihrer dem Pumpengehäuse 20 abgewandten Seite direkt mit der im Flüssigkeitspfad 2 geführten Flüssigkeit beaufschlagt ist, welche im Betrieb vom Insulinreservoir (nicht gezeigt) kommend über die Zuführungsöffnung 14a in den Innenraum 12 einströmt und diesen über die Abführungsöffnung 14b in Richtung zum Patienten verlässt. Wie erkennbar ist, trägt die Membran 7 im vorliegenden Fall einen starren Reflektorkörper 11, welcher zusammen mit der Membran 7 und dem Gehäuse des Adapters 21 durch mehrfachen Spritzguss hergestellt wurde und an seiner dem Pumpengehäuse 20 zugewandten Fläche durch Bedampfen mit einer Verspiegelung versehen wurde und dadurch eine Reflektorfläche bildet.

Wie in Zusammenschau mit Fig. 2b ersichtlich ist, welche eine Ansicht des in Fig. 2a dargestellten Bereichs der Anordnung aus Sicht der durch die Linie A-A in Fig. 2a definierten Schnittebene zeigt und der Verdeutlichung der Position der einzelnen Elemente 4, 5, 7, 11 zueinander dient, ist die Membran 7 bei dieser Ausführungsform oval ausgebildet und der Reflektor 11 aussermittig auf dieser angeordnet, mit dem Effekt, dass sich der Reflektor 11 und damit auch die mit der Lichtquelle 4 angestrahlte Reflektorfläche desselben bei einer Verwölbung der Membran 7 neigt und dadurch den Einfalls- und den Ausfallswinkel des Lichtstrahls verändert, so dass es zu einer Verlagerung des reflektierten Lichtpunktes auf dem X-Y-Sensor 5 kommt, aus welcher auf die Verwölbung der Membran 7 geschlossen werden kann. Das Gehäuse des Adapters 21, die Membran 7 und der obere Teil des Insulinpumpengehäuses 20 sind im vorliegenden Fall aus einem lichtundurchlässigen Kunststoff hergestellt, um einen Einfall von Störlicht in den Bereich zwischen dem Reflektor der Membran 7 und den Messmitteln 4, 5 und damit die Möglichkeit einer Fehlmessung auszuschliessen.

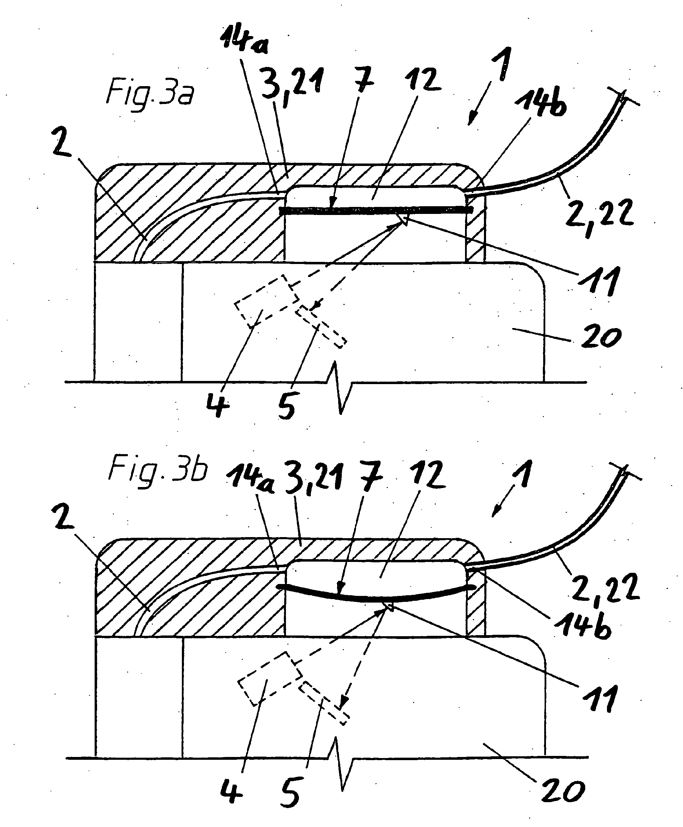

Die Figuren 3a und 3b zeigen Darstellungen wie Fig. 2a einer zweiten erfindungsgemässen Ausführungsform der Erfindung, und zwar einmal bei im wesentlichen drucklosen Flüssigkeitspfad 2 (Fig. 3a), was dem Zustand im Normalbetrieb der Insulinpumpe 1 entspricht, und einmal bei stark druckbeaufschlagtem Flüssigkeitspfad 2 (Fig. 3b), was einem Zustand entspricht, bei dem der Flüssigkeitspfad 2 patientenseitig durch eine Okklusion verschlossen ist. Wie in Fig. 3b gut zu erkennen ist, verwölbt sich infolge des Überdrucks im Innenraum 12 die Membran 7 in Richtung zum Pumpengehäuse 20 hin, wodurch der aussermittig auf der Membran 7 angeordnete Reflektorkörper 11 schräggestellt wird und seine Reflektorfläche den Lichtstrahl auf einen anderen Sektor des X-Y-Sensors 5 lenkt. Im Gegensatz zu der in den Figuren 2a und 2b dargestellten Ausführungsform weist hier die Reflektorfläche infolge der keilförmigen Ausbildung des Reflektorkörpers 11 immer einen Winkel zur Oberfläche der Membran 7 auf, weshalb es hier möglich ist, die Lichtquelle 4 und den Sensor 5 seitlich versetzt zur Membran 7 im Gehäuse 20 der Insulinpumpe 1 anzuordnen.

Wie für den Fachmann ohne Weiteres zu erkennen ist, kann durch gezielte Platzierung, Anordnung und Formgebung der Reflektorfläche die Sensitivität und Präzision signifikant beeinflusst werden. So kann z.B. durch eine geeignete Geometrie die Grösse des reflektierten Lichtpunktes verändert werden, ein nichtlineares Verhalten der Membran 7 korrigiert werden oder eine progressive oder degressive Verschiebung des Ausgangssignals des Sensors 5 in Abhängigkeit des Fluiddruckes erreicht werden. Wie dies im Einzelfall geschehen kann, ist dem Fachmann bekannt und soll deswegen hier nicht weiter erläutert werden.

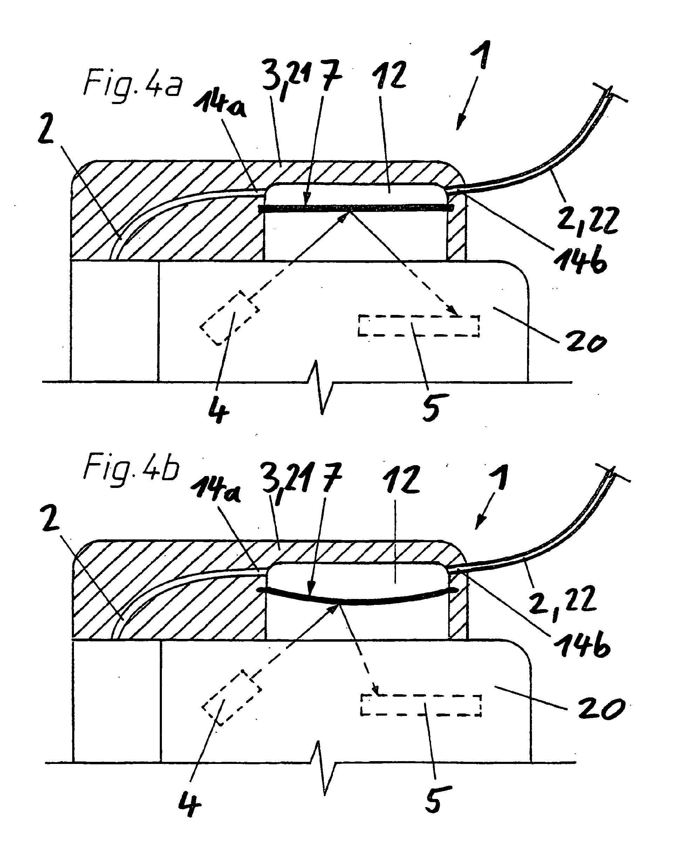

Die Figuren 4a und 4b zeigen Darstellungen wie die Figuren 3a und 3b einer weiteren bevorzugten Ausführungsform der Erfindung, welche sich von der in den Figuren 2a und 2b gezeigten Ausführungsform im wesentlichen dadurch unterscheidet, dass die Membran 7 keinen starren Reflektorkörper 11 trägt, sondern selbst die Reflektorfläche bildet. Dabei ist nur ein kleiner, zur Reflektion benötigter Teilbereich der Membrane 7 verspiegelt, der übrige Bereich ist zur Vermeidung von Streulicht satiniert bzw. mattiert. Auch hier ist es vorgesehen, die Geometrie der Spiegelfläche, wie zuvor für die Ausführungsformen mit starren Reflektionskörpern 11 beschrieben, zwecks Erzielung bestimmter Effekte mit einer bestimmten, von einer planen Fläche abweichenden Grundform zu versehen, was beispielsweise durch Verwendung einer im drucklosen Zustand bereits verwölbten Membran oder auch durch entsprechendes Ausformen lediglich des zur Reflexion verwendeten Teilbereichs einer ansonsten im drucklosen Zustand planen Membran erfolgen kann.

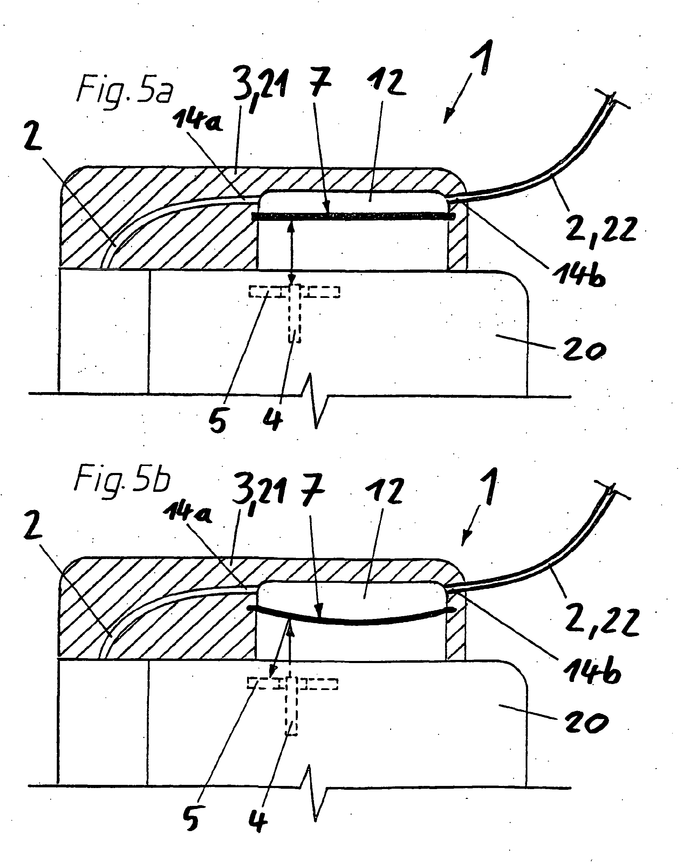

Die Figuren 5a und 5b zeigen Darstellungen wie die Figuren 4a und 4b noch einer weiteren bevorzugten Ausführungsform der Erfindung, welche sich von der in den zuvor erwähnten Figuren gezeigten Ausführungsform dadurch unterscheidet, dass hier die Lichtquelle 4 und der Sensor 5 eine kombinierte Einheit bilden, bei welcher die Lichtquelle 4 mittig angeordnet ist und von einer kreisringförmigen photosensitiven Sensorfläche 5 umgeben ist. Wie zu erkennen ist, wird bei dieser Ausführungsform der Lichtstrahl derartig zur Reflektorfläche der Membran 7 geführt, dass er im drucklosen Zustand zur Lichtquelle 4 zurückreflektiert wird und nicht auf den Sensor 5 auftrifft. Da der auftreffende Lichtstrahl hier in einem Bereich ausserhalb des Membranenzentrums reflektiert wird, führt die in Fig. 5b gezeigten Verwölbung der Membran 7 infolge eines Druckaufbaus im Flüssigkeitspfad 2 und damit im Innenraum 12 des Adapters 21 zu einer Neigung der Reflektorfläche und damit zu einem Auftreffen des reflektierten Lichtstrahls auf den Sensor 5, wodurch die Druckänderung detektierbar wird.

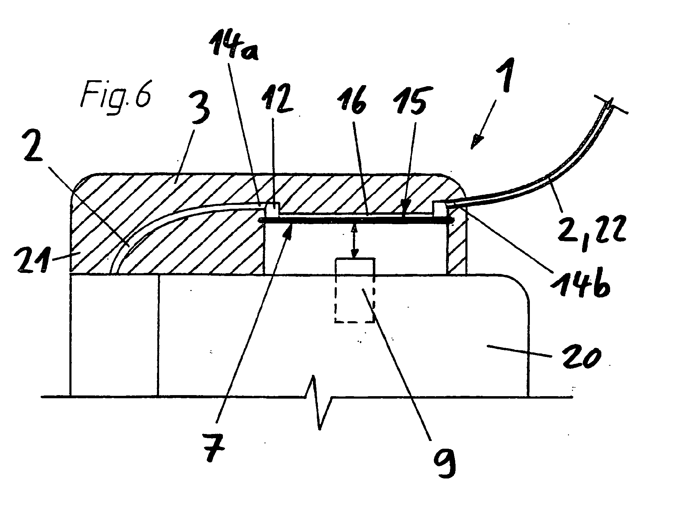

Fig. 6 zeigt eine Darstellung wie Fig. 2a noch einer weiteren, nicht erfindungsgemässen Ausführungsform, welche sich von der in Fig. 5a gezeigten dadurch unterscheidet, dass auf der dem Insulinpumpengehäuse 20 abgewandten Seite der Membran 7, also im Innenraum 12 des Adapterteils 21, eine Kontaktfläche 15 aus mehreren parallel in Strömungsrichtung des Flüssigkeitspfades 2 verlaufenden Stützrippen 16 vorhanden ist, an welcher die Membran 7 bei drucklosem Flüssigkeitspfad 2 bzw. Innenraum 12 anliegt und dadurch eine definierte Position einnimmt. Auch wird hierdurch das Volumen des Innenraumes 12 und damit das "Totvolumen" des Adapters 21 deutlich herabgesetzt. Die gerippte Ausbildung der Kontaktfläche 15 ermöglicht einen problemlosen Durchfluss der Flüssigkeit und verhindert zudem ein Ankleben der Membran 7 an der Kontaktfläche 15. Zudem weist die hier gezeigte Anordnung weder eine Lichtquelle noch einen photosensitiven Sensor auf, basiert also nicht auf einem optischen Messverfahren, sondern weist einen kommerziell erhältlichen Ultraschall-Abstandssensor 9 auf, mittels welchem die Distanz zur infusionspumpenseitigen Oberfläche der Membran 7 im Zentrum der Membran 7 gemessen wird.

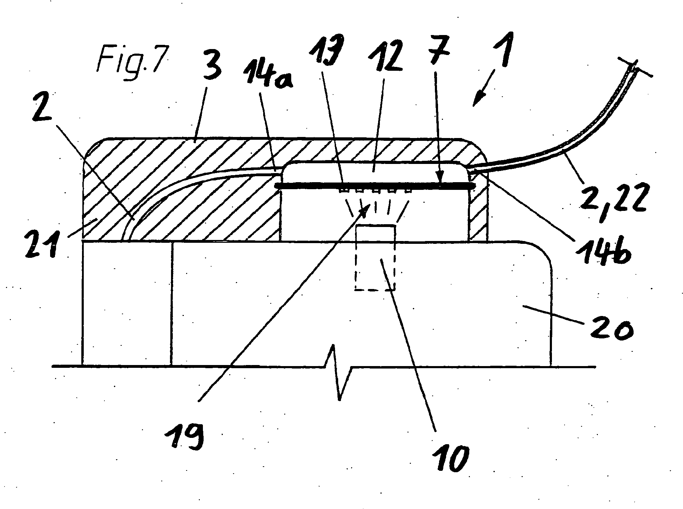

Fig. 7 zeigt eine Darstellung wie Fig. 2a noch einer weiteren nicht erfindungsgemässen Ausführungsform, welche sich von der in Fig. 5a gezeigten dadurch unterscheidet, dass auf der dem Insulinpumpengehäuse 20 zugewandten Seite der Membran 7 mehrere zueinander beabstandete, zu einem Strichmuster 19 gruppierte Elemente 13 angeordnet sind, welche sich farblich von der eigentlichen Membranfläche abheben und deren Abstand zueinander mittels einer digitalen Kamera 10 mit Auswerteelektronik gemessen wird. Verwölbt sich die Membran 7 infolge einer Druckänderung im Raum 12, so dehnt sich die Membran 7 und die Abstände der Elemente 13 zueinander verändern sich, was mittels der Kamera 10 mit Auswerteelektronik festgestellt und zur Erkennung der Druckänderung verwendet wird.

Bei den in den Figuren 2a bis 7 gezeigten Ausführungsformen ist es auch vorgesehen, dass die Membran 7 einstückig mit dem diese tragenden Gehäuseteil des Adapters 21 ausgebildet ist, z.B. durch gleichzeitige Herstellung der Membran 7 und Adaptergehäuses oder zumindest des die Membran 7 tragenden Teils des Adaptergehäuses aus einem Kunststoffmaterial durch Spritzgiessen in einem einzigen Arbeitsgang. Dabei sind auch Ausführungen vorgesehen, bei denen der Adapter 21 von einem einstückig mit der Membran 7 ausgebildeten ersten Bauteil und einem weiteren Bauteil gebildet wird, indem diese Bauteile unter Bildung des Flüssigkeitspfades 2 und des Innenraums 12 zusammengefügt werden, z.B. mittels Verkleben oder Verschweissen, insbesondere Laserschweissen, gegebenenfalls auch unter Verwendung zusätzlicher Dichtelemente.

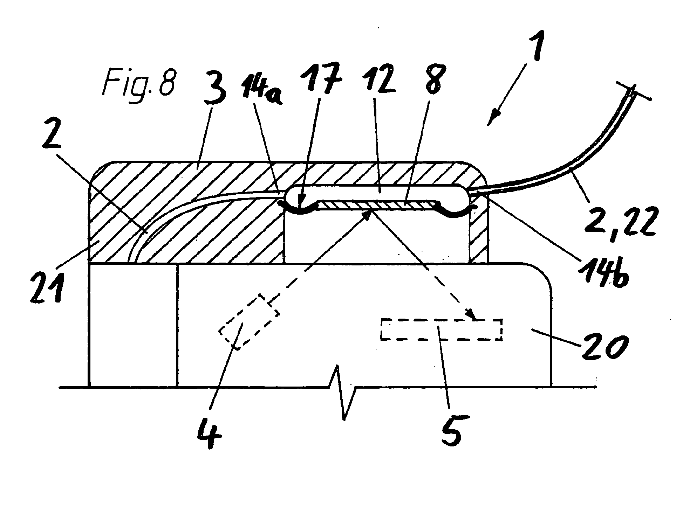

Fig. 8 zeigt eine Darstellung wie Fig. 2a noch einer weiteren erfindungsgemässe Ausführungsform, welche sich von der in Fig. 4a gezeigten lediglich dadurch unterscheidet, dass anstelle der Membran 7 eine mittels eines umlaufenden sickenartigen Elements 17 geführte starre Scheibe 8 vorhanden ist, welche sich bei Druckänderung translatorisch in Richtung zum Pumpengehäuse 20 hin bzw. von diesem weg verschiebt und an welcher der von der Lichtquelle 4 ausgesendete Lichtstrahl zum Sensor 5 hin reflektiert wird. Die Scheibe 8 ist einstückig mit dem Sickenelement 17 und mit dem Gehäuse des Adapters 21 ausgebildet, was durch mehrfaches Spritzgiessen erreicht wurde.

Wie bereits eingangs erwähnt wurde, sind bei den zuvor beschriebenen Insulinpumpen 1 die anspruchsgemässen Körperlichkeiten 3, welche die zu messende Gestaltänderung bei einer Änderung des Flüssigkeitsdrucks im Flüssigkeitspfad erfahren, von dem als Einwegartikel ausgebildeten Infusionsset-Adapter 21 gebildet, während die eigentlichen Messmittel 4, 5, 9, 10 bestehend entweder aus Lichtquelle 4 und Sensor 5, Ultraschallsensor 9 oder Kamera 10, dauerhaft und ohne Werkzeug nichtentfernbar im Gehäuse 20 der Insulinpumpe 1 angeordnet sind und über Jahre hinweg im Einsatz stehen. Der Wechsel des Infusionssets inklusive Adapter 21 erfolgt hingegen in Intervallen von wenigen Tagen und ist ohne Werkzeug möglich. Nach einem solchen Wechsel wird vorgängig zur eigentlichen Messung ein sogenannter Nullabgleich vorgenommen, indem bei drucklosem Flüssigkeitspfad 2 das individuelle Signal der Messmittel im drucklosen Zustand der Messanordnung ermittelt und als Basis für die weitere Messung verwendet wird. In der Folge wird sodann zur Ermittlung des Drucks bzw. der Druckänderung lediglich die Änderung gegenüber diesem Basissignal gemessen, also eine Relativmessung durchgeführt. Hierdurch ergibt sich der Vorteil, dass die individuellen Fertigungs- und Füge- bzw. Positionierungstoleranzen praktisch eliminiert werden können, so dass eine relativ grosszügige Tolerierung der Einwegkomponenten möglich wird und hier eine beträchtliche Kosteneinsparung in Aussicht genommen werden kann.

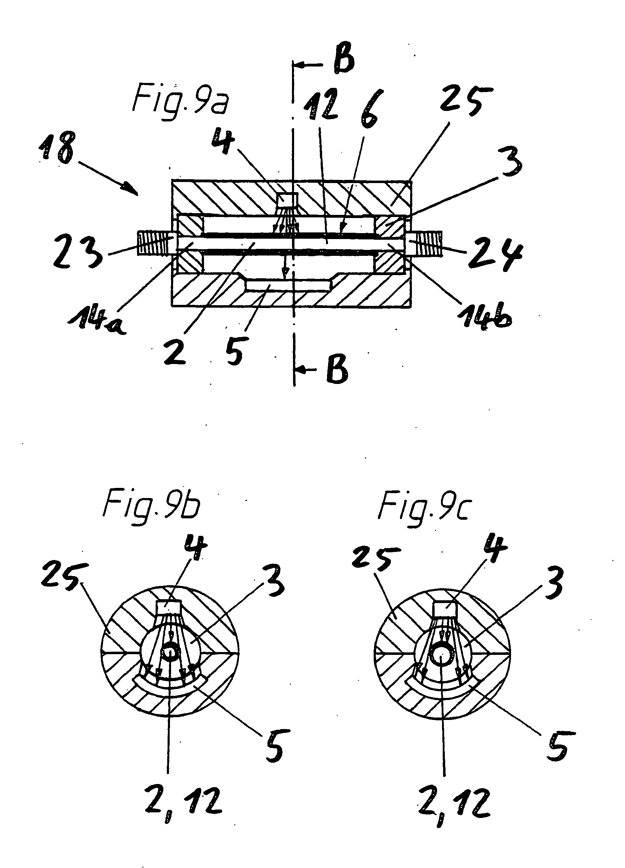

Fig. 9a zeigt einen Längsschnitt durch eine nicht erfindungsgemässe Vorrichtung 18 zur Erkennung einer Druckänderung im Flüssigkeitspfad 2 einer Mikrodosiervorrichtung, welche im Gegensatz zu den zuvor gezeigten Vorrichtungen nicht in eine Mikrodosiervorrichtung integriert ist, sondern eine eigenständige Funktionseinheit darstellt, welche in den Flüssigkeitspfad 2 einer Mikrodosiervorrichtung, in einen Bypass dazu oder in einen mit dem Flüssigkeitspfad druckmässig wirkverbundenen Blindkanal eingekoppelt werden kann, um dann als eigenständiges Gerät den Druck im Flüssigkeitskanal 2 zu überwachen und gegebenenfalls Alarmmeldungen, z.B. auf akustischem und/oder optischem Wege, abzugeben, falls ein unzulässiger Druck bzw. eine unzulässige Druckänderung ermittelt wird. Hierzu umfasst die Vorrichtung 18 ein elastisches Schlauchstück 6, welches zwischen zwei Anschlussstücken 23, 24 mit Anschlussgewinden zum Einkoppeln in den Flüssigkeitspfad 2 einer Mikrodosiervorrichtung angeordnet ist und eine anspruchsgemässe Körperlichkeit 3 bildet, bei welcher die Flüssigkeit über die Zuführungsöffnung 14a in den Innenraum 12 des Schlauchstückes 6 eintritt und diesen über die Abführungsöffnung 14b wieder verlässt, wobei sich der Durchmesser des Schlauchstückes 6 in Abhängigkeit von dem im Innenraum 12 des Schlauchstücks 6 herrschenden Flüssigkeitsdruck ändert. Dabei wird das Schlauchstück 6 mit den beiden Anschlussstücken 23, 24 von einem zweiteiligen Gehäuse 25 lichtdicht unter Bildung eines zylindrischen Innenraums umschlossen, in dessen Begrenzungswandungen an einer ersten Position eine Lichtquelle 4 und an einer zweiten, der Lichtquelle 4 gegenüberliegenden Position ein sich gewölbt entlang der Wandung erstreckender photosensitiver X-Y-Sensor 5 angeordnet sind, derart, dass sich der Schlauchabschnitt 6 zwischen diesen befindet und die Sensorfläche teilweise beschattet.

Wie aus den Figuren 9b und 9c erkennbar wird, ist dieser Schatten bei der in Fig. 9b dargestellten Situation, in welcher der Flüssigkeitspfad 2 und damit der Schlauchabschnitt 6 drucklos ist, deutlich kleiner als in der in Fig. 9c dargestellten Situation mit infolge einer Okklusion druckbeaufschlagtem Flüssigkeitspfad 2, da der Durchmesser der Schlauchabschnitts 6 mit zunehmendem Druck grösser wird. Diese Veränderung der Grösse des Schattenbildes des Schlauchabschnitts 6 wird durch den Sensor 5 in Kombination mit einer in der Vorrichtung 18 enthaltenen Auswerteelektronik (nicht gezeigt) detektiert, in einen Druckwert umgerechnet und mit einem zuvor abgespeicherten Vorgabewert verglichen, bei dessen Überschreitung von der Vorrichtung 18 ein akustisches Alarmsignal ausgesandt wird. Anstelle des X-Y-Sensors 5 wäre hier auch eine einfache Photozelle denkbar, deren abgegebene Spannung sich bei einer Änderung ihrer beleuchteten Fläche verändert.

Auch wenn bei den hier gezeigten Beispielen eine ovale Membran gezeigt wurde, sind auch kreisrunde oder eckige, insbesondere viereckige und quadratische Membranen vorgesehen.

Als Werkstoffe für die mit der Flüssigkeit in Kontakt stehenden Bauteile wie Gehäuseteile, Membranen, Schlauchabschnitte usw. sind thermoplastische Kunststoffe wie Polyäthylen (PE LD, PE HD), Polypropylen (PP), Cycloolefin-Copolymer (COC), Copolyester (PET, PETG, PCTG, PCTA), Acrilnitril-Butadien-Styrol (ABS), Polymethylmethacrylat (PMMA), Fluor-Ethylen-Polymer (FEP) bevorzugt. Weitere mögliche Kunststoffe sind Acrylnitril Butadien Styrol (ABS), Butylkautschuk (IIR), Flüssigkristall-Polymer (LCP), Polybutylen Terephthalat (PBT), Polycarbonat (PC), Glycol modifiziertes Polycyclohexylendimethylen Terephthalat (PCTG), Polyetheretherketon (PEEK), Polyethylen Terephthalat (PET), Polyethylen (PE), Polyethylen Terephthalat modifiziert mit Cyclohexandimethanol (PETG), Polymethylmethacrylat (PMMA), Polypropylen (PP), Polystyrol (PS), Polytetrafluorethylen (PTFE), Polyurethan (PU bzw. PUR), Styrol Acrylnitril (SAN), Thermoplastisches Polyurethan (TPUR) und Zyklisches Olefin Copolymer (COC). Zudem ist es bevorzugt, die mit dem Fluid in Kontakt stehenden Flächen zu beschichten, um optimale und auf das durchströmende Medium angepasste Eigenschaften zu erreichen, insbesondere betreffend Benetzbarkeit, Strömungsverhalten, Diffusionsverhalten, Adsorption, etc.

Die Membranen können zudem auch aus Metallfolien oder Verbundfolie aus Kunststoff und Metall, insbesondere Aluminium gebildet sein.

Geeignete Lichtquellen für die hier verwendeten optischen Messverfahren stellen beispielsweise LEDs mit verschiedenen Wellenlängen (Infrarot bis Ultraviolett), Laserdioden, Blitzleuchten, Entladungslämpchen, Glühbirnen etc. dar, wobei die abgegebene Strahlung auch durch Lichtleiter umgelenkt, mit Linsen gebündelt und/oder durch Blenden begrenzt sein kann.

Anstelle der gezeigten Durchlaufanordnungen ist es zudem vorgesehen, solche Vorrichtungen als Blindkanalausführungen mit lediglich einer Öffnung zum Innenraum 12 auszubilden bzw. bei den gezeigten Anordnungen eine der Öffnungen 14a, 14b zu verschliessen.

Zudem ist es auch vorgesehen, die gezeigten Anordnungen (ausgenommen die in Fig. 6 gezeigte) zusätzlich oder ausschliesslich zum Detektieren eines Unterdrucks im Flüssigkeitspfad einer Mikrodosiervorrichtung zu verwenden, z.B. im Flüssigkeitspfad zwischen dem Flüssigkeitsreservoir und der Förderpumpe. Hierdurch lässt sich beispielsweise ein leeres Flüssigkeitsreservoir oder eine Okklusion in diesem Bereich des Flüssigkeitspfades erkennen.

## Patentansprüche

1. Vorrichtung zur Durchführung eines Verfahrens zur Erkennung einer Druckänderung im Flüssigkeitspfad (2) einer Mikrodosiervorrichtung (1) für flüssige Stoffe, insbesondere für flüssige Medikamente, mit folgenden Verfahrensschritten:
Bereitstellen einer Körperlichkeit (3, 21), welche derartig ausgestaltet und mit der im Flüssigkeitspfad (2) geführten Flüssigkeit wirkverbunden ist, dass sie bei Änderung des Drucks der im Flüssigkeitspfad (2) geführten Flüssigkeit eine Gestaltänderung erfährt und
Detektieren der Gestaltänderung der Körperlichkeit (3, 21) mittels berührungsloser Messmittel (4, 5, 9, 10) zwecks Erkennung der Druckänderung,
wobei zum Detektieren der Gestaltänderung ein Lichtstrahl an einer sich bei Druckänderung verlagernden und/oder verformenden Fläche reflektiert wird, umfassend
a) eine Körperlichkeit (3, 21) mit einem flüssigkeitsdichten Innenraum (12), welcher über mindestens eine Öffnung (14a, 14b) mit dem Flüssigkeitspfad (2) einer Mikrodosiervorrichtung (1) verbunden oder verbindbar ist, wobei die Körperlichkeit (3, 21) derartig ausgestaltet ist, dass sie bei einer Änderung des Druckes der im Betrieb im Flüssigkeitspfad (2) der Mikrodosiervorrichtung (1) geführten Flüssigkeit eine Gestaltänderung erfährt, und
b) Messmittel (4, 5, 9, 10), welche derartig ausgestaltet und mit der Körperlichkeit (3, 21) gekoppelt sind, dass mit ihnen eine Gestaltänderung der Körperlichkeit (3, 21) infolge einer Änderung des Druckes der Flüssigkeit im Flüssigkeitspfad (2) berührungslos detektierbar ist,
**dadurch gekennzeichnet, dass** die Messmittel (4, 5, 9, 10) ausgestaltet sind zum Detektieren einer Lage- oder Formänderung einer sich bei Druckänderung verlagernden und/- oder verformenden Fläche der Körperlichkeit (3, 21) mittels Reflexion eines Lichtstrahls an der sich verlagernden oder verformenden Fläche auf einen photosensitiven Sensor (5), insbesondere einen Punkt-, Linien- oder X-Y-Sensor (5).

2. Vorrichtung nach Anspruch 1, wobei der Innenraum (12) der Körperlichkeit (3, 21) einen Teil des Flüssigkeitspfades (2), zumindest einen Teil einer Bypassleitung zum Flüssigkeitspfad (2) oder zumindest einen Teil eines mit dem Flüssigkeitspfad (2) kommunizierenden Blindkanals bildet.

3. Vorrichtung nach einem der Ansprüche 1 bis 2, wobei die Vorrichtung derartig ausgestaltet ist, dass ein werkzeugloser Wechsel der Körperlichkeit (3, 21) bei an der Vorrichtung verbleibenden Messmitteln (4, 5, 9, 10) möglich ist.

4. Vorrichtung nach einem der Ansprüche 1 bis 3, wobei die Körperlichkeit (3, 21) einen elastischen Schlauchabschnitt (6) oder einen elastischen blasenartigen Körper umfasst, welcher bei einer Änderung des Drucks der in seinem Innenraum (12) befindlichen Flüssigkeit eine Dehnung oder Schrumpfung, insbesondere eine Durchmesserveränderung, erfährt.

5. Vorrichtung nach einem der Ansprüche 1 bis 4, wobei die Körperlichkeit (3, 21) eine Membran (7) umfasst, welche sich bei einer Änderung des Drucks der in seinem Innenraum (12) befindlichen Flüssigkeit mehr oder weniger stark verwölbt.

6. Vorrichtung nach Anspruch 5, wobei die Membran (7) einen reflektierenden Bereich aufweist oder einen insbesondere starren Reflektor (11) trägt, dessen Form- oder Lageänderung infolge einer Druckänderung der Flüssigkeit mit den Messmitteln (4, 5, 9, 10) durch Reflexion eines Lichtstrahls an demselben detektierbar ist.

7. Vorrichtung nach Anspruch 6, wobei der den reflektierenden Bereich oder den Reflektor (11) umgebende Bereich den Membran (7) satiniert ist.

8. Vorrichtung nach einem der Ansprüche 6 bis 7, wobei die Membran (7) und insbesondere die gesamte Körperlichkeit (3, 21) aus einem lichtundurchlässigen Material ist.

9. Vorrichtung nach einem der Ansprüche 5 bis 8, wobei die Membran (7) auf einer ihrer beiden Seiten direkt mit der Flüssigkeit beaufschlagt bzw. beaufschlagbar ist.

10. Vorrichtung nach Anspruch 9, wobei die Membran (7) in einem Zustand, in welchem die Flüssigkeit im Flüssigkeitspfad (2) drucklos ist, auf ihrer flüssigkeitsbeaufschlagten Seite an einer Kontaktfläche (15) anliegt, und insbesondere, wobei diese Kontaktfläche (15) eine Oberflächenstruktur, insbesondere mit Kerben, Rippen (16) oder einem Pyramidenmuster aufweist.

11. Vorrichtung nach einem der Ansprüche 1 bis 10, wobei die Körperlichkeit (3, 21) ein starres Bauteil (8) umfasst, welches sich bei einer Änderung des Druckes der in seinem Innenraum (12) befindlichen Flüssigkeit gegenüber einem anderen Element der Körperlichkeit (3, 21) verschiebt, verdreht oder neigt.

12. Vorrichtung nach Anspruch 11, wobei die Körperlichkeit (3, 21) eine Kolben-Zylinder-Anordnung mit einem insbesondere federkraftbelasteten Kolben umfasst, welcher bei Änderung des Flüssigkeitsdrucks eine Verschiebung im Zylinder erfährt.

13. Vorrichtung nach Anspruch 11, wobei die Körperlichkeit (3, 21) ein durch ein sickenförmiges Bauteil (17) verschiebbar geführtes kolben- oder plattenförmiges Element (8) umfasst, welches bei Änderung des Flüssigkeitsdrucks eine Verschiebung oder Schrägstellung erfährt.

14. Vorrichtung nach einem der Ansprüche 12 bis 13, wobei der Kolben oder das kolben- oder plattenförmige Element (8) auf einer Seite direkt mit der Flüssigkeit beaufschlagt oder beaufschlagbar ist.

15. Vorrichtung nach einem der Ansprüche 1 bis 14, wobei die Messmittel (4, 5, 9, 10) ausgestaltet sind zum kontinuierlichen, intervallweisen oder ereignisgesteuerten Detektieren der Gestaltänderung.

16. Vorrichtung nach einem der Ansprüche 1 bis 15, wobei die Vorrichtung eine Auswertungselektronik aufweist, mit welcher eine detektierte Gestaltänderung oder ein aus dieser Gestaltänderung abgeleiteter Druckwert mit mindestens einem insbesondere benutzerprogrammierbaren Vorgabewert verglichen werden kann und bei Über- oder Unterschreiten des Vorgabewertes ein Alarm ausgelöst werden kann, welcher insbesondere akustisch, optisch und/oder taktil wahrnehmbar gemacht werden kann.

17. Vorrichtung nach einem der Ansprüche 1 bis 16, wobei die Vorrichtung eine Auswertungselektronik aufweist, mit welcher eine detektierte Gestaltänderung bzw. ein daraus abgeleiteter Druckwert, insbesondere vor dem Vergleichen mit einem Vorgabewert, mittels eines Korrekturalgorithmus um den Einfluss bestimmter Störgrössen, insbesondere um den Einfluss des Umgebungsluftdruckes, der Umgebungstemperatur, mechanischer Positionsabweichungen zwischen der Körperlichkeit (3, 21) und den Messmitteln (4, 5, 9, 10) und/oder um den Einfluss von Vibrationen korrigiert werden kann.

18. Vorrichtung nach einem der Ansprüche 1 bis 17, wobei die Vorrichtung eine eigenständige Funktionseinheit (18) ist oder in eine Mikrodosiervorrichtung (1), insbesondere für flüssige Medikamente, integriert ist.

19. Mikrodosiervorrichtung (1), insbesondere zur zeitlich gesteuerten Abgabe von flüssigen Medikamenten, mit einer Vorrichtung nach einem der Ansprüche 1 bis 18, wobei die Körperlichkeit (3, 21) als werkzeuglos austauschbares Wechselteil ausgebildet ist und die Messmittel (4, 5, 9, 10) werkzeuglos unentfernbar an der Mikrodosiervorrichtung (1) angeordnet sind.

20. Mikrodosiervorrichtung (1) nach Anspruch 19 zur zeitlich gesteuerten Zuführung von Insulin zum Körper einer Person mittels eines mit der Mikrodosiervorrichtung (1) verbundenen Infusionssets (21, 22), wobei die Messmittel (4, 5, 9, 10) zur Mehrfachverwendung in das Pumpengehäuse (20) integriert sind und die Körperlichkeit (3, 21) zur Einmalverwendung Teil des Infusionsset-Adapters (21) bzw. des Infusionssets (21, 22) ist.

## Claims

1. A device for carrying out a method for detecting a pressure change in the liquid path (2) of a micro-dosing device (1) for liquid substances, in particular for liquid medicaments, having the following method steps:
providing a body (3, 21) which is designed and functionally coupled to the liquid that is carried in the liquid path (2) in such a way that it undergoes a change in form when the pressure of the liquid that is carried in the liquid path (2) changes, and
detecting the change in form of the body (3, 21) by means of contactless measuring means (4, 5, 9, 10) for the purpose of detecting the pressure change,
wherein, in order to detect the change in form, a light beam is reflected at a surface that changes position and/or deforms when the pressure changes, comprising
a) a body (3, 21) having a liquid-tight interior compartment (12), which is connected, or can be connected to the liquid path (2) of a micro-dosing device (1) by means of at least one opening (14a, 14b), wherein the body (3, 21) is designed in such a way that it undergoes a change in form when the pressure of the liquid carried during operation in the liquid path (2) of the micro-dosing device (1) changes, and
b) measuring means (4, 5, 9, 10) that are designed and coupled to the body (3, 21) in such a way that a change in form of the body (3, 21) as a result of a change in pressure of the liquid in the liquid path (2) can be detected by them without contact,
**characterized in that** the measuring means (4, 5, 9, 10) are designed to detect a change in position or form of a surface of the body (3, 21), which changes in position and/or deforms when the pressure changes, by reflection of a light beam at the surface that changes in position or deforms onto a photo-sensitive sensor (5), in particular a point, line or X-Y sensor (5).

2. The device according to claim 1, wherein the interior compartment (12) of the body (3, 21) forms part of the liquid path (2), at least part of a bypass line to the liquid path (2), or at least part of a blind channel communicating with the liquid path (2).

3. The device according to one of claims 1 to 2, wherein the device is designed in such a way that the body (3, 21) can be changed without tools while the measuring means (4, 5, 9, 10) remain on the device.

4. The device according to one of claims 1 to 3, wherein the body (3, 21) comprises an elastic tube section (6) or an elastic bladder-like body, which undergoes expansion or shrinkage, in particular a diameter change, when the pressure of the liquid in its interior compartment (12) changes.

5. The device according to one of claims 1 to 4, wherein the body (3, 21) comprises a membrane (7), which bulges to a greater or lesser extent when the pressure of the liquid in its interior compartment (12) changes.

6. The device according to claim 5, wherein the membrane (7) has a reflecting area or carries particularly a rigid reflector (11), the change in form or position of which as a result of a pressure change of the liquid can be detected by the measuring means (4, 5, 9, 10) by the reflection of a light beam at said reflecting area or reflector.

7. The device according to claim 6, wherein the area of the membrane (7) surrounding the reflecting area or the reflector (11) is satined.

8. The device according to one of claims 6 to 7, wherein the membrane (7) and in particular the whole body (3, 21) is made of an opaque material.

9. The device according to one of claims 5 to 8, wherein on one of its two sides the membrane (7) is directly acted upon, or can be acted upon, by the liquid.

10. The device according to claim 9, wherein, in a state in which the liquid in the liquid path (2) is not pressurized, the side of the membrane (7) which is acted upon by the liquid rests on a contact surface (15), and in particular wherein this contact surface (15) has a surface structure, in particular with grooves, ribs (16), or a pyramid pattern.

11. The device according to one of claims 1 to 10, wherein the body (3, 21) comprises a rigid component (8) that moves, twists or tilts with respect to another element of the body (3, 21) when the pressure of the liquid in its interior compartment (12) changes.

12. The device according to claim 11, wherein the body (3, 21) comprises a piston-cylinder arrangement with a piston that in particular is spring-loaded, and which undergoes a displacement in the cylinder when the liquid pressure changes.

13. The device according to claim 11, wherein the body (3, 21) comprises a piston- or plate-shaped element (8) that is movably guided by a corrugation-shaped component (17), and which undergoes a displacement or inclination when the liquid pressure changes.

14. The device according to one of claims 12 to 13, wherein the piston or the piston- or plate-shaped element (8) on one side is directly acted upon, or can be acted upon, by the liquid.

15. The device according to one of claims 1 to 14, wherein the measuring means (4, 5, 9, 10) are designed for detecting the form change continuously, at intervals, or controlled by events.

16. The device according to one of claims 1 to 15, wherein the device has evaluation electronics with which a detected form change, or a pressure value derived from this form change, can be compared with at least one in particular user-programmable setpoint value, and with which, if the value exceeds or goes below the setpoint value, an alarm can be triggered, which can be made perceptible in particular acoustically, optically and/or in a tactile manner.

17. The device according to one of claims 1 to 16, wherein the device has evaluation electronics with which a detected change in form, or a pressure value derived therefrom, can be corrected by means of a corrective algorithm, in particular before the comparison with a setpoint value, by the influence of certain disturbance variables, in particular by the influence of the ambient air pressure, the ambient temperature, mechanical position deviations between the body (3, 21) and the measuring means (4, 5, 9, 10), and/or by influence of vibrations.

18. The device according to one of claims 1 to 17, wherein the device is an independent functional unit (18), or is integrated in a micro-dosing device (1), in particular for liquid medicaments.

19. A micro-dosing device (1), in particular for the time-controlled dispensing of liquid medicaments, with a device according to one of claims 1 to 18, wherein the body (3, 21) is designed as an interchange part that can be exchanged without tools, and the measuring means (4, 5, 9, 10) are arranged on the micro-dosing device (1) in such a way that they cannot be removed without tools.

20. The micro-dosing device (1) according to claim 19 for the time-controlled delivery of insulin into the body of a person by means of an infusion set (21, 22) that is connected to the micro-dosing device (1), wherein the measuring means (4, 5, 9, 10) for multiple use are integrated in the pump housing (20), and the body (3, 21) for single use is part of the infusion set adapter (21), or the infusion set (21, 22) respectively.

## Revendications

1. Dispositif pour la mise en oeuvre d'un procédé de détection d'une variation de pression dans le trajet de liquide (2) d'un dispositif de microdosage (1) pour substances liquides, en particulier pour médicaments liquides, comprenant les étapes de procédé suivantes :
fournir un corps (3, 21) qui est configuré et qui coopère avec le liquide conduit dans le trajet de liquide (2) de telle manière qu'il connaisse une variation de sa forme en cas de variation de la pression du liquide conduit dans le trajet de liquide (2), et
détecter la variation de la forme du corps (3, 21) à l'aide de moyens de mesure sans contact (4, 5, 9, 10) afin de détecter la variation de pression,
dans lequel, dans le but de détecter la variation de forme, un faisceau lumineux est réfléchi sur une surface qui se déplace et/ou qui se déforme en cas de variation de la pression,
comprenant:
a) un corps (3, 21) comportant un espace intérieur (12) étanche au liquide, qui est relié ou est apte à être relié par au moins une ouverture (14a, 14b) avec le trajet de liquide (2) d'un dispositif de microdosage (1), le corps (3, 21) étant conçu pour connaître une variation de sa forme en cas de variation de la pression du liquide conduit lors du fonctionnement dans le trajet de liquide (2) du dispositif de microdosage (1), et
b) des moyens de mesure (4, 5, 9, 10) qui sont conçus et couplés avec le corps (3, 21) afin de permettre de détecter sans contact une variation de la forme du corps (3, 21) à la suite d'une variation de la pression du liquide dans le trajet de liquide (2),
**caractérisé par le fait que** les moyens de mesure (4, 5, 9, 10) sont conçus pour détecter une variation de position ou de forme d'une surface du corps (3, 21) qui se déplace et/ou qui se déforme en cas de variation de la pression au moyen d'une réflexion d'un faisceau lumineux sur la surface qui se déplace ou qui se déforme sur un capteur photosensible (5), en particulier un capteur ponctuel, linéaire ou X-Y (5).

2. Dispositif selon la revendication 1, dans lequel l'espace intérieur (12) du corps (3, 21) forme une partie du trajet de liquide (2), au moins une partie d'une conduite de dérivation du trajet de liquide (2) ou au moins une partie d'une conduite fermée qui communique avec le trajet de liquide (2).

3. Dispositif selon l'une des revendications 1 à 2, dans lequel le dispositif est conçu de manière à permettre un changement sans outil du corps (3, 21) alors que les moyens de mesure (4, 5, 9, 10) restent sur le dispositif.

4. Dispositif selon l'une des revendications 1 à 3, dans lequel le corps (3, 21) comprend un segment de flexible élastique (6) ou un corps élastique en forme de vessie qui, en cas de variation de la pression du liquide qui se trouve dans son espace intérieur (12), connaît une expansion ou une rétraction, en particulier une variation du diamètre.

5. Dispositif selon l'une des revendications 1 à 4, dans lequel le corps (3, 21) comprend une membrane (7) qui se gonfle plus ou moins fortement en cas de variation de la pression du liquide qui se trouve dans son espace intérieur (12).

6. Dispositif selon la revendication 5, dans lequel la membrane (7) présente une zone réfléchissante ou porte un réflecteur (11), en particulier rigide, dont la variation de forme ou de position à la suite d'une variation de la pression du liquide est apte à être détectée à l'aide des moyens de mesure (4, 5, 9, 10) par la réflexion d'un faisceau lumineux sur celui-ci.

7. Dispositif selon la revendication 6, dans lequel la zone de la membrane (7) qui entoure la zone réfléchissante ou le réflecteur (11) est satinée.

8. Dispositif selon l'une des revendications 6 et 7, dans lequel la membrane (7) et en particulier la totalité du corps (3, 21) est faite d'un matériau opaque.

9. Dispositif selon l'une des revendications 5 à 8, dans lequel la membrane (7) est ou est apte à être exposée directement au liquide par l'une de ses deux faces.

10. Dispositif selon la revendication 9, dans lequel, dans un état où le liquide se trouvant dans le trajet de liquide (2) n'est pas sous pression, la membrane (7) repose par sa face baignée par le liquide contre une surface de contact (15) et, en particulier, dans lequel cette surface de contact (15) présente une structure superficielle, en particulier comportant des encoches, des nervures (16) ou un motif de pyramides.

11. Dispositif selon l'une des revendications 1 à 10, dans lequel le corps (3, 21) comprend un composant rigide (8) qui, en cas de variation de la pression du liquide qui se trouve dans son espace intérieur (12), se décale, se tourne ou s'incline par rapport à un autre élément du corps (3, 21).

12. Dispositif selon la revendication 11, dans lequel le corps (3, 21) comprend un mécanisme à piston et cylindre avec un piston qui est en particulier chargé par l'effet d'un ressort et qui, en cas de variation de la pression du liquide, connaît un déplacement dans le cylindre.

13. Dispositif selon la revendication 11, dans lequel le corps (3, 21) comprend un élément (8) en forme de piston ou de plaque qui est guidé en coulissant par un composant en forme de moulure (17) et qui, en cas de variation de la pression du liquide, connaît un déplacement ou un placement en biais.

14. Dispositif selon l'une des revendications 12 à 13, dans lequel le piston ou l'élément (8) en forme de piston ou de plaque est ou est apte à être exposé directement au liquide par une face.

15. Dispositif selon l'une des revendications 1 à 14, dans lequel les moyens de mesure (4, 5, 9, 10) sont conçus pour détecter la variation de la forme de manière continue, intermittente ou commandée par des événements.

16. Dispositif selon l'une des revendications 1 à 15, dans lequel le dispositif comprend une électronique d'analyse grâce à laquelle une variation détectée de la forme ou une valeur de pression dérivée de cette variation de la forme peut être comparée avec au moins une valeur prédéterminée, en particulier qui peut être programmée par l'utilisateur, et dans lequel, en cas de dépassement ou de soupassement de la valeur prédéterminée, une alarme peut être déclenchée, qui peut en particulier être rendue perceptible de manière acoustique, optique et/ou tactile.

17. Dispositif selon l'une des revendications 1 à 16, dans lequel le dispositif comprend une électronique d'analyse grâce à laquelle une variation détectée de la forme ou une valeur de pression qui en est dérivée, en particulier avant la comparaison avec une valeur prédéterminée, peut être corrigée à l'aide d'un algorithme de correction pour éliminer l'influence de certaines grandeurs perturbatrices, en particulier l'influence de la pression de l'air environnant, de la température environnante, des écarts mécaniques de position entre le corps (3, 21) et les moyens de mesure (4, 5, 9, 10), et/ou l'influence de vibrations.

18. Dispositif selon l'une des revendications 1 à 17, dans lequel le dispositif est une unité fonctionnelle autonome (18) ou bien est intégré dans un dispositif de microdosage (1), en particulier pour médicaments liquides.

19. Dispositif de microdosage (1), en particulier pour l'administration pilotée dans le temps de médicaments liquides, comportant un dispositif selon l'une des revendications 1 à 18, dans lequel le corps (3, 21) est réalisé sous la forme d'une pièce de rechange pouvant être remplacée sans outils et les moyens de mesure (4, 5, 9, 10) sont montés sur le dispositif de microdosage (1) de manière à ne pas pouvoir être déposés sans outils.

20. Dispositif de microdosage (1) selon la revendication 19, destiné à l'administration pilotée dans le temps d'insuline dans le corps d'une personne, à l'aide d'un ensemble de perfusion (21, 22) raccordé avec le dispositif de microdosage (1), dans lequel les moyens de mesure (4, 5, 9, 10) sont intégrés dans le corps de pompe (20) en vue d'un usage multiple et le corps (3, 21) est un élément à usage unique de l'adaptateur d'ensemble de perfusion (21) ou de l'ensemble de perfusion (21, 22).
